# EUROPEAN PATENT SPECIFICATION

(11) **EP 0 401 266 B1**
(45) Date of publication and mention of the grant of the patent: **05.05.1993**
(21) Application number: 89902838.5
(22) Date of filing: 16.02.1989
(51) Int. Cl.: A61M 25/00, B08B 9/02

(54) **CATHETER CLEANER**
REINIGUNGSSYSTEM FÜR KATHETER
SYSTEME DE NETTOYAGE DE CATHETER

(30) Priority: 19.02.1988 SE 8800578
(43) Date of publication of application: 12.12.1990
(73) Proprietor: LÖVQVIST, Bo, S-804 26 Gävle (SE)
(72) Inventor: LÖVQVIST, Bo, S-804 26 Gävle (SE)
(74) Representative: Hopfgarten, Nils
(86) International application number: SE8900067
(87) International publication number: WO8907466

(56) References cited:
- FR-A- 2 479 035
- US-A- 723 745
- US-A- 1 965 128
- US-A- 3 958 573

## Description

The present invention relates to a combined catheter cleaner and catheter arrangement set out in the prior art portion of claim 1.

In difficult urination, e.g. due to an enlarged prostate gland (prostatism) catheters are used to empty the bladder, until the trouble has been remedied by an operation. Prostatism is usual among older men, and the Swedish health service uses a large number, of the order of magnitude 900.000 catheters of different kinds. The catheters used consist of a rubber tube of a certain stiffness which is inserted through the urethra into the bladder.

A problem causing large costs for the healt service, and suffering for the patient, is that clogging occurs in the catheters.

So far, it has been attempted to dissolve blockages formed in this way in the catheter by injecting a saline solution into it. However, this method is not very effective, and the catheter must usually be exchanged when a blockage occurs. Catheter exchanges are painful to the patient and must be carried out by medically trained healthcare personnel, such as doctors or nurses, and results in large costs. A catheter exchange thus costs between 500 and 1.000 Crowns.

Another attempt to reduce the problem with clogging in the catheter is by using a larger catheter. This increases the discomfort caused to the patient, and only delays the formation of a blockage in the catheter.

The object of the present invention is to eliminate the above-mentioned problem.

This object is achieved with a catheter cleaner according to claim 1.

There is accordingly achieved by the present invention a catheter and catheter cleaner, the use of which is so simple that the patient himself, a relative, a "home help" or the like can clean the catheter regularly, e.g. once a week, thus to avoid blockages with accompanying catheter exchanges. With the catheter cleaner in accordance with the invention, catheter cleaning can thus take place in the home without the assistance of specially, medically trained personnel, as well as in a hospital or other health care institution.

In addition, the catheter cleaner and catheter in accordance with the invention are simple and cheap to produce and signify a surprisingly cheap solution, seen altogether, to a problem affecting a very large number of people. Health care costs decrease for these people, their work performance increases, they acquire greater confidence in social situations and in working life, and the psychological and physical suffering in connection with prostatism decreases in general. The risk of urine drops is reduced by the invention, and cheaper catheters can be used.

Since the catheter and catheter cleaner in accordance with the invention are cheap and simple to manufacture, they are produced for throw-away use and is supplied in a sterile package.

As the risk of the catheter clogging has been eliminated with the present invention, it is also possible to use thinner catheters, which reduces the patient's discomfort.

A very essential property of the cathether and cleaner according to the invention is that the cleaner means is formed such that it cannot be inserted "too far" into the catheter so that it could reach into the bladder. The cleaner means according to the invention is for this reason provided with stop means which, by engagement against the catheter, stop any further insertion of the cleaner means when it has arrived at its completely inserted position.

The increased thickness of the outer portion of the cleaner means can be achieved by applying a plastic tube on the outside of the outer portion of the cleaner means. Further increased safety against insertion of the cleaner means too far is thus obtained, and a better grip on the grip part is obtained for rotating the cleaner means.

From FR-A-2 479 035, US-A-723 745 and US-A-1 965 128 pipe or outlet cleaners are known, having a gripping part at one end and the other end being moved to clean said pipe or outlet, while being rotated or moved axially. However, none of these means are useful for the purpose of cleaning a radial opening of a catheter. Thus FR-A-2 479 035 discloses a cleaner to be moved in an axial direction in a pipe or outlet to be cleaned. US-A-723 745 discloses a cleaning means being built in sections and having a cleaning head which is rotatively symmetrical, not suitable for use in catheter cleaning. US-A-1 965 128 discloses a cleaning means with scraping or cutting elements, which are spring biased against the inner wall of the tube for the purpose of scraping or cutting away incrustations or deposits on the inner wall of the tube. It would be impossible to insert such a cleaning means in a catheter in the urethra.

Embodiments of the catheter and the catheter cleaner in accordance with the invention, selected as examples, will now be described in more detail in connection with the accompanying drawings, where
Figure 1 schematically illustrates a prior art catheter cleaner inserted into a catheter.
Figure 2 illustrates the inner end portion of a prior art cleaner means.
Figure 3 illustrates a catheter cleaner inserted in a catheter in accordance with the invention.
Figure 4 illustrates the use of the embodiment according to Figure 3.
Figure 5 illustrates the catheter cleaner according to the invention.

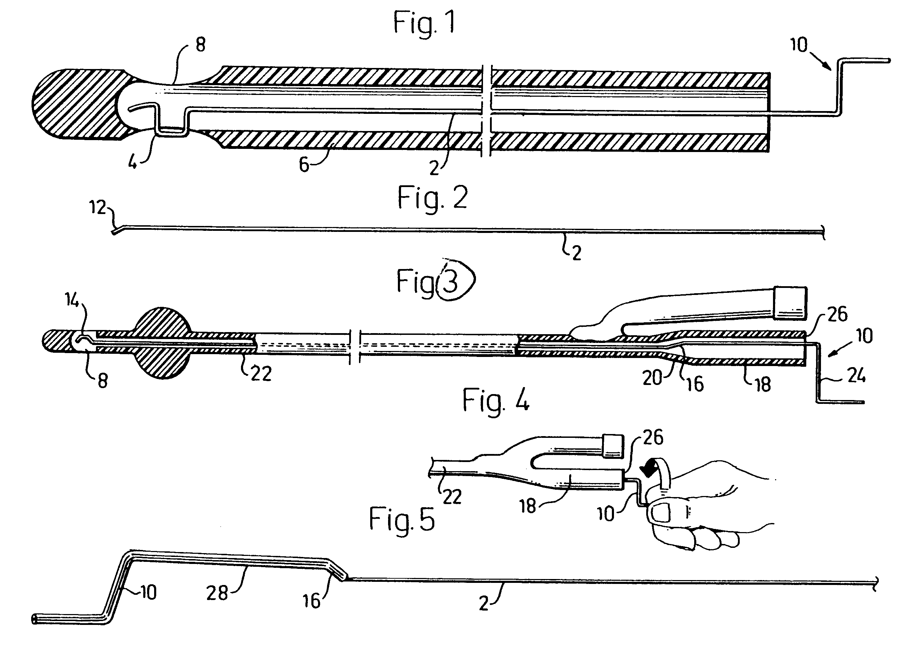

Figure 1 schematically illustrates an embodiment of a catheter cleaner inserted in a catheter. The catheter cleaner comprises a filament 2 of comparatively stiff material, but with a certain flexibility for enabling insertion into the catheter also when this is curved during use. A suitable filament material may be a PVC plastics.

The end portion of the filament 2 is formed into a bent configuration 4, which cleans the region of the opening 8 of the catheter 6 when the cleaner means is inserted inside the catheter. The region of the opening is particularly subjected to blockage by salts in the urine crystallising and forming deposits there.

At the outer portion of the cleaner means there is a gripping part in the form of a crank 10 formed for rotating the filament 2 around its axis, see also Figure 4. Rotation of the cleaner means thus provides cleaning the catheter 6 and effective cleaning of the opening 8 by the bent configuration 4.

It is essential that the cleaner means is formed such that the risk for insertion too far thereof is eliminated, since otherwise the cleaner means could penetrate into the bladder, where damage could then occur. According to Figure 1, the bent configuration 4 will serve as stop means preventing further insertion of the cleaner means, as soon as the bent configuration reaches the area of the catheter opening 8.

In Figure 2 there is illustrated an inner portion of the cleaner means for cleaning the opening region of the catheter. The end portion 12 of the filament 2 is simply bent to a given angle. The bent part 12 serves to effectively clean the opening region when the cleaner means is rotated.

The catheter cleaner inserted in a conventional kind of catheter in accordance with the invention is illustrated in Figure 3. The cleaner means is provided at its inner end portion with a bent configuration 14 for cleaning the catheter opening 8 by rotating the cleaner means by the crank 10.

According to Figure 3, the cleaner means is formed with a further bent configuration 16 which can only be inserted in the expanded connection portion 18 of the catheter, and serves as a stop against too far insertion of the cleaner means by abutting against the catheter wall in the transition region 20 between the expanded connection portion 18 and the catheter 22 itself.

In addition, the gripping part formed as a crank 10 is disposed such that the crank part 24 also serves as a stop against too far insertion of the cleaner means into the catheter by abutting against the catheter end 26.

In Figure 3, there is thus triple security against too far insertion of the cleaner means, by the cleaning configuration 14 in the catheter opening 8, the bent configuration 16 and the crank 10 which function as three stops.

The use of the catheter according to Figure 3 is illustrated in Figure 4 by rotating the cleaner means using the crank 10.

The cleaner means of the catheter cleaner in accordance with the invention is further illustrated in Figure 5, the outer portion of the cleaner means from the bent configuration 16 and outwards having been given a greater thickness, by having a plastic tube 28 applied on the outside of the filament 2. By this thickening or enlargement of the outer part of the cleaner means, there is achieved further security against too far insertion of the cleaner means into the catheter, since the enlarged part 28 cannot be inserted in the catheter itself. The crank part 10 will also be easier to grip, due to its increased thickness.

## Claims

1. Catheter cleaner and catheter, comprising an elongate cleaner means being formed for insertion into a catheter passed through the urethra, and being formed with a gripping part at one end for cleaning the catheter by rotation of the cleaner means about its longitudinal axis, said cleaner comprising a filament (2) of comparatively stiff material, but with a certain flexibility for enabling insertion thereof also when said catheter is curved, said filament being formed with the gripping part (10) at its outer end and a bent insertion tip portion (12,14) at its opposite end (12,14), said filament being so formed that with the cleaner means inserted in the catheter, said bent insertion tip portion situated opposite the gripping part of the filament is located at the opening (8) of the catheter passed through the urethra for cleaning said catheter opening by rotating the filament, **characterized in** that the innermost end of the gripping part of the filament (2) is bent in such a manner and at such a distance from its insertion tip (12,14) that the bent portion (16) of the gripping part can be inserted in an expanded outer connection portion (18) of the catheter, and serves as a stop against too far insertion of said cleaner means by abutting against the catheter wall in the transition region (20) between the expanded connection portion and the catheter (22) itself, and in that an outer portion (28) of the cleaner means from the bent portion (16) and outwards has a larger thickness than the portion of said cleaner means situated inside the outer connection portion (18,20).

2. Catheter according to claim 1, **characterized in** that a plastics tube (28) is applied on the gripping part (10), i.e. outside of the filament (2) of the cleaner means to give this portion a larger thickness.

## Patentansprüche

1. Katheter-Reinigungsvorrichtung und Katheter, mit einem langgestreckten Reinigungselement, welche für das Einsetzen in einen durch die Urethra geführten Katheter geeignet ist, und mit einem Griffteil an einem Ende versehen ist, welcher zum Reinigen des Katheters durch Drehen des Reinigungselements um seine Längsachse dient, wobei die Reinigungsvorrichtung eine Faser (2) aus vergleichsweise steifem Material aufweist, welches jedoch eine gewisse Flexibilität aufweist, um das Einsetzen der Faser auch dann zu gestatten, wenn der Katheter gekrümmt ist, wobei die Faser mit dem Griffteil (10) an ihrem äußerem Emde und einem umgebogenen Einsetzspitze (12,14) an dem gegenüberliegenden Ende (12,14) versehen ist, und die Faser so geformt ist, daß bei in den Katheter eingesetztem Reinigungselement die gebogene Einsetzspitze der Faser, welche dem Griffteil gegenüberliegend angeordnet ist, an der Öffnung (8) des durch die Urethra eingesetzten Katheters angeordnet ist, um die Öffnung des Katheters durch Drehen der Faser zu reinigen zu können, dadurch gekennzeichnet, daß das innerste Ende des Griffteils der Faser (2) derart und in einem solchen Abstand von der Einsetzspitze (12,14) gebogen ist, daß der gebogene Teil (16) des Griffteils in einen aufgeweiteten äußeren Verbindungsteil (18) des Katheters eingesetzt werden kann und als Anschlag gegen ein zu weites Hereinschieben des Reinigungselements dient, indem der gebogene Teil gegen die Katheterwand in dem Übergangsbereich (20) zwischen dem aufgeweiteten Verbindungsteil und dem Katheter (22) selbst anschlägt, und daß ein äußerer Teil (28) des Reinigungselements von dem gebogenen Teil (16) und von diesem aus auswärts eine größere Dicke als derjenige Teil des Reinigungselements aufweist, welcher bezogen auf den äußeren Verbindungsteil (18,20) einwärts angeordnet ist.

2. Katheter nach Anspruch 1, dadurch gekennzeichnet, daß ein Kunststoffrohr (28) auf den Griffteil (10) aufgebracht ist, d.h. auf die Außenseite der Faser (2) des Reinigungselements, um diesem Teil eine größere Dicke zu geben.

## Revendications

1. Dispositif de nettoyage de cathéter et cathéter correspondant, comportant des moyens de nettoyage allongés constitués pour s'engager dans un cathéter introduit dans l'urètre et agencés avec une partie de manoeuvre à une extrémité pour nettoyer le cathéter par rotation des moyens de nettoyage autour de leur axe longitudinal, ledit dispositif de nettoyage comportant un fil (2) en matériau relativement rigide mais présentant une certaine flexibilité pour permettre son introduction même quand ledit cathéter est incurvé, ledit fil étant façonné avec la partie de manoeuvre (10) à sont extrémité extérieure et avec un bout d'introduction coudé (12, 14) à son extrémité opposée (12, 14), ledit fil étant façonné de façon telle que lorsque les moyens de nettoyage sont engagés dans le cathéter, ledit bout d'introduction coudé, situé à l'opposé de la partie de manoeuvre du fil, soit placé dans l'embouchure (8) du cathéter introduit dans l'urètre pour nettoyer ladite embouchure du cathéter par rotation du fil, caractérisés en ce que l'extrémité intérieure extrême de la partie de manoeuvre du fil (2) est coudée de façon telle et à une distance telle du bout d'introduction (12, 14) que la partie coudée (16) de la partie de manoeuvre peut être introduite dans une partie de liaison extérieure élargie (18) du cathéter, en servant de butée d'arrêt contre l'engagement trop profond desdits moyens de nettoyage par butée contre la paroi du cathéter dans la zone de transition (20) entre la partie de liaison élargie et le cathéter (22) proprement dit, et en ce qu'une partie extérieure (28) des moyens de nettoyage comprenant la partie coudée (16) et la partie située vers l'extérieur, a une plus grande épaisseur que la partie desdits moyens de nettoyage située vers l'intérieur à partir de la partie extérieure de liaison et de transition (18, 20).

2. Cathéter selon la revendication 1, caractérisé en ce qu'un tube en matière plastique (28) est appliqué sur la partie de manoeuvre (10), c'est-à-dire sur la partie extérieure du fil (2) des moyens de nettoyage, pour donner une plus grande épaisseur à ce cette partie.
